# EUROPÄISCHE PATENTANMELDUNG

(11) **EP 1 310 257 A2**
(43) Veröffentlichungstag der Anmeldung: **14.05.2003**
(21) Anmeldenummer: 03002577.9
(22) Anmeldetag: 03.09.1998
(51) Int. Cl.: A61K 31/565, A61K 31/57, A61P 15/18

(54) **Kombination zur hormonalen Kontrazeption**

(30) Priorität: 11.09.1997 DE 19739916
(62) Teilanmeldung aus: 98954135.4
(71) Anmelder: HESCH, Rolf Dieter, D-78464 Konstanz (DE)
(72) Erfinder: HESCH, Rolf Dieter, D-78464 Konstanz (DE)
(74) Vertreter: Goddar, Heinz J., Dr.

(57) **Zusammenfassung**

Verwendung einer Kombination aus Levonorgestrel und Ethinylestradiol als alleinigen hormonellen Wirkstoffen zur Herstellung eines Kontrazeptionsmittels zur kontinuierlichen Ovulationshemmung und Unterdrückung des Menstruationszyklus durch ununterbrochene orale, transdermale oder Depot-Verabreichung.

## Beschreibung

Die Erfindung betrifft die Verwendung einer Kombination von nur zwei Hormonkomponenten, nämlich eines einzelnen Gestagens und eines einzelnen Estrogens, als alleinigen hormonellen Wirkstoffen, zur Herstellung eines Kontrazeptionsmittels zur Ovulationshemmung und ggf. zur Prophylaxe der Osteoporose und/oder zur Behandlung des prämenstruellen Syndroms.

Seit Beginn der Verfügbarkeit hormoneller Kontrazeptiva in den 60er Jahren wurde eine Vielzahl von hormonellen Komponenten hinsichtlich ihrer Eignung in den verschiedensten Darreichungsschemata untersucht. Grundsätzlich ist eine Einteilung in Kombinations- und Sequenzpräparate möglich.

Bei bekannten Kombinationspräparaten wird bespielsweise, sofern die gewünschte Zyklusdauer 28 Tage beträgt, 21 Tage lang in konstanter oder wechselnder absoluter und/oder relativer Dosierung eine Kombination aus einem Estrogenpräparat und einem Gestagenpräparat verabreicht, wobei das Estrogenpräparat z.B. natürliches Estrogen oder synthetisches Ethinylestradiol sein kann, und sich an die Einnahme der vorgenannten 21 Tageseinheiten eine siebentägige Pause anschließt, in der es zu einer die natürliche Monatsblutung simulierenden Entzugsblutung kommt.

Bei den bekannten Sequenzpräparaten wird, wiederum bei einer gewünschten Zyklusdauer von 28 Tage, 7 Tage lang ein reines Estrogenpräparat und dann 15 Tage lang eine Kombination aus einem Estrogenpräparat und einem Gestagenpräparat verabreicht, wobei sich auch hier wieder eine einnahmefreie Zeit von z.B. 6 Tagen anschließt, in der es zur Entzugsblutung kommt. Es ist zwar bereits bekannt, die den Kombinations- und dem Sequenzpräparaten eigenen Einnahmepausen im Interessen einer größeren Einnahmesicherheit dadurch zu überbrükken, daß innerhalb der betreffenden Tage Placebos verabreicht werden, jedoch ist man bisher stets davon ausgegangen, daß während der etwa einwöchigen Einnahmepause keine Hormone der hier in Rede stehenden Art verabreicht werden dürfen, um eine zuverlässige Entzugsblutung zu gewährleisten. Lediglich bei Substitutionspräparaten in der Menopause der älteren Frauen hat man während des Gesamtzyklus Hormone verabreicht, beispielsweise in der Abfolge 10 Tage Estrogenpräparat, 11 Tage Kombination aus Estrogen- und Gestagenpräparat, 7 Estrogenpräparat, 7 Estrogenpräparat in besonders niedriger Dosierung, jedoch sind diese Substitutionspräparate zur Ovulationshemmung nicht geeignet.

Die bei Substitutionstherapie verwendeten Sequenzpräparate sind zur Kontrazeption insbesondere deshalb ungeeignet, weil das natürliche Estradiol in der gegebenen Dosierung die Ovulation nicht verhindert und die Phase, in der Gestagen verabreicht wird, mit nur 11 Tagen zu kurz ist. Die vorstehend beschriebene sequentielle Anordnung gewährleistet bei den Substitutionspräparaten jedoch eine relativ gute Zykluskontrolle.

In der EP 0 309 263 A1 ist ein Kontrazeptionsmittel beschrieben, welches im wesentlichen aus Blöcken von Estrogen-Gestagen-Kombinationen besteht, wobei jeder Block entweder Estrogen- oder Gestagen-Dominanz aufweist, und während 21 - 24 Tagen des 28-Tage-Zyklus der Frau eingenommen wird. An die vorgenannte Hormon-Verabreichungsphase von 21 - 24 Tagen schließt sich zwingend eine 4 - 7 Tage umfassende hormonfreie Einnahmepause an, wobei während dieser hormonfreien Tage beispielsweise ein Placebo verabreicht werden kann. Durch die zwingend vorgeschriebene Einnahmepause wird eine Abbruchblutung bewirkt, die bei der Vorgehensweise nach der EP 0 309 263 A1 als zwingend notwendig angesehen wird.

Aus der EP 0 628 312 A1 ist ein Kombinationspräparat zur Kontrazeption bekannt, welches aus einer oder mehreren Stufen besteht. Dabei ist vorgesehen, daß mindestens eine Stufe die Kombination von drei Komponenten, nämlich einem biogenen Estrogen, einem synthetischen Estrogen und einem Gestagen enthält und die weiteren Stufen jeweils aus einem pharmazeutisch unbedenklichen Placebo oder einem biogenen oder synthetischen Gestagen, oder einem biogenen oder synthetischen Estrogen, oder einer Kombination aus zwei Komponenten, nämlich einem biogenen Estrogen, einem synthetischen Estrogen und einem Gestagen oder einer Kombination aus synthetischen Estrogen und einem Gestagen, bestehen. Mit dem darin beschriebenen Stufenkonzept erfolgt typischerweise eine Zustandsänderung über die Zeit. Eine derartige Zustandsänderung kann sowohl dergestalt erfolgen, daß sich die Zusammensetzung der die Stufe ausbildenden Phasen hinsichtlich der eingesetzten Komponenten verändert, als auch dadurch, daß sich lediglich die Konzentrationen der in den die Stufe ausbildenden Phasen eingesetzten Komponenten ändern. Aus der Gesamtoffenbarung der vorgenannten Druckschrift, einschließlich sämtlicher Beispiele, geht hervor, daß dort von der bis dahin als unerläßlich angesehenen Praxis nicht abgegangen werden soll, durch Gestagenentzug eine Abbruchblutung zu bewirken.

Auch bei einer aus der DE-OS 44 05 898 bekannten Vorgehensweise wird die Unterdrückung des Menstruationszyklus nicht in Erwägung gezogen. Die Verwendung eines Estrogens und eines Gestagens in transdermaler Applikation zur Kontrazeption wird zwar diskutiert, jedoch werden in diesem Zusammenhang ausdrücklich die Zyklusregulierung und die Zyklusstabilisierung angesprochen.

Schließlich ist in der nicht vorveröffentlichten, aber prioritätsälteren EP 0 911 029 A2 eine Kontrazeptionsbehandlung beschrieben, bei der eine Kombination aus Estrogen und Gestagen, nämlich etwa 5 - 35 µg Ethinylestradiol und etwa 0,025 bis 10 mg Norethindronacetat, für bis zu 110 aufeinander folgender Tage monophasisch verabreicht werden, jedoch ist auch hier zwingend eine daran anschließende Einnahmepause von 3 bis 10 Tagen vorgeschrieben, um eine Abbruchblutung hervorzurufen, ehe dann ein neuer Hormon-Einnahmezyklus beginnt.

Aus Contraception 51, Seiten 355 - 358, veröffentlicht im Jahr 1995, ist ferner eine Versuchsbeschreibung bekannt, bei der einer Gruppe von Patientinnen über einen Zeitraum von einem Jahr jeweils für 21 Tage eine tägliche Dosis von 0,25 mg Levonorgestrel und 0,05 mg Ethinylestradiol, also eine sehr hohe Dosis, gefolgt von einer siebentägigen Einnahmepause, verabreicht wurden, während eine Vergleichsgruppe für den gesamten Jahreszeitraum die vorgenannte Hormonkombination täglich ohne Einnahmeunterbrechung erhielt, resultierend in einer deutlich höheren Hormonverabreichung auf Monatsbasis bei der letztgenannten kontinuierlichen Vergleichsgruppe im Verhältnis zu der erstgenannten. Die Hormonverabreichung erfolgte bei beiden Gruppen ausschließlich vaginal, wobei hinsichtlich einer zusätzlichen Kontrollgruppe, bei der die Verabreichung oral erfolgte, ausdrücklich festgestellt ist, daß sich hierbei im Vergleich zur vaginalen Verabreichung keine Unterschiede zeigten. Die Untersuchung ergab, daß bei der kontinuierlichen Verabreichung der vorgenannten Hormonkomponenten in erheblichem Maße Zwischenblutungen auftraten, so daß die Akzeptanz der dort beschriebenen Vorgehensweise vor allem bei Frauen des westlichen Kulturkreises - die in der vorgenannten Veröffentlichung diskutierten Untersuchungen wurden in einer Drittweltumgebung vorgenommen - nicht gegeben ist und die vorgenannte Versuchsbeschreibung eher Anlaß geben könnte, von einer kontinuierlichen Verabreichung der beschriebenen Hormonkombination abzusehen und stattdessen monatliche Zwangs-Einnahmepausen vorzusehen.

Aus einem Artikel in American Journal of Hematology 52, 237, veröffentlicht im Jahr 1996, ist ein Vorschlag bekannt, für drei Monate ein orales Kontrazeptionsmittel, welches aus 150 mg Levonorgestrel und 30 µg Ethinylestradiol, also auch hier eine recht hohe Ethinylestradioldosis, zu verabreichen, wobei dies zur Behandlung einer Patientin mit kongenitaler Afibrinogenemie diente, um auf diese Weise sowohl eine Menstruation zu verhindern als auch eine Ovulation zu unterdrücken. In dem Artikel heißt es, daß die vorgeschriebene Kontrazeptionsbehandlung besser kontinuierlich erfolgen soll, um bei Patientinnen mit kongenitaler Afibrinogenemie und dem daraus resultierenden Blutverlust infolge von Menstruation eine Amenorrhö zu beheben. Es handelt sich hierbei um die Verwendung eines Kontrazeptionsmittels zur Behandlung einer spezifischen Erkrankung, für die exzessive Menstruationsblutungen typisch sind, wobei aber selbst zu diesem Zweck keine kontinuierliche Hormonverabreichung für einen längeren Zeitraum als drei Monate empfohlen wird.

Insgesamt ist festzustellen, daß es bis zum Vorschlag gemäß der nicht vorveröffentlichten DE-OS 197 05 229, bei der allerdings zwingend die Verwendung von drei Hormonkomponenten zur hormonalen Kontrazeption zur Behandlung und/oder Prophylaxe von Tumoren der Brustdrüsen vorgesehen ist, als unerläßlich angesehen wurde, eine Abbruchblutung herbeizuführen, während eine kontinuierliche Verabreichung von zwei Komponenten, nämlich einem Estrogen und einem Gestagen, wie z.B. in der DE-PS 44 05 591 beschrieben, lediglich als zur postmenopausalen Hormonsubstitution geeignet angesehen wurde.

Der Erfindung liegt die Aufgabe zugrunde, ein Mittel zur hormonalen Kontrazeption bereitzustellen, welches eine hohe kontrazeptive Sicherheit gewährleistet und, Zwischenblutungen ausschließt. Weiterhin sollen die ansonsten bei hormonalen Mitteln zur Kontrazeption beobachteten Nebenwirkungen weiter reduziert werden.

Erfindungsgemäß wird diese Aufgabe durch die Merkmalskombination des Patentanspruches 1 gelöst.

Besonders bevorzugte Ausführungsformen der Erfindung sind Gegenstand der Unteransprüche.

Durch die erfindungsgemäße kontinuierliche und kombinierte Verabreichung eines Mittels, bestehend aus zwei Hormonkomponenten, nämlich Levonorgestrel und Ethinylestradiol, kann eine hohe kontrazeptive Sicherheit erreicht werden.

Unter kontinuierlicher Verabreichung wird hierin eine über den Anwendungszeitraum ununterbrochene Verabreichung verstanden, bei der keine bezüglich der Hormonkomponenten einnahmefreien Intervalle vorgesehen sind. Dies bedeutet auch, daß eine Unterbrechung der Verabreichung des Mittels durch Gabe von Placebos anstelle des hormonalen Mittels nicht vorgesehen ist. In der Folge kommt es über die gesamte, typischerweise die Zeitspanne mehrerer Monate bis Jahre umfassenden Verabreichungsdauer zu keinen Veränderungen der grundsätzlichen Zusammensetzung der Hormonkomponenten. Vielmehr werden über die gesamte Verabreichungsdauer ununterbrochen und unverändert die das erfindungsgemäße hormonale Mittel ausbildenden Hormonkomponenten in unveränderter Konzentration verabreicht. Denkbar ist jedoch, daß die Konzentration von Estrogen, verstanden in der gesamten hierin definierten Breite des Begriffs, und Gestagen, ebenfalls verstanden in der gesamten hierin definierten Breite des Begriffs, bei älteren Frauen gegenüber jüngeren verändert wird. Dies kann auch so geschehen, daß entlang der kontinuierlichen Applikation zunächst mit einer bestimmten Zusammensetzung begonnen wird und diese dann im Laufe von Wochen, Monaten und Jahren an die geänderten biologischen Bedürfnisse der Frau durch die Applikation eines Folgepräparates angepaßt wird, das jedoch auch ein Mittel gemäß der vorliegenden Erfindung umfaßt.

Infolge der kontinuierlichen Applikation der genannten Hormonkomponenten wird gewährleistet, daß die natürlicherweise im weiblichen Organismus ablaufenden hormonalen Vorgänge die kontrazeptive Sicherheit nicht durchbrechen.

Durch die Estrogen-Komponente respektive durch spezifische Wirkung von hydrophoben Ringsubstanzen mit estrogenartiger Wirkung kann es zu einer Suppression der Gonadoprotine kommen. Dies ist erwünscht. Die daraus resultierende Unterdrückung der Ovarialfunktion wird ausgeglichen durch eine adäquate Substitution von Estrogenwirkung. Damit kommt es zu einer Verhütung der Entwicklung einer Osteoporose, die günstigen Gefäßwirkungen von Estrogenen bleiben erhalten, ebenso wird der Lipidostoffwechsel nicht ungünstige beeinflußt. Durch Unterbrechung der zyklusabhängigen Instabilität im Hormonsystem kann das prämenstruelle Syndrom günstig beeinflußt werden, darüber hinaus wird die Homöostase des Gerinnungssystems nicht gestört, da das labile Gleichgewicht, in welchem sich das Gerinnungssystem befindet, nicht durch das Auf und Ab der Hormonschwankungen aktiviert und desaktiviert wird. Deshalb eignet sich das hormonale Mittel nach der Erfindung insbesondere auch für Frauen im Alter von mehr als 40 Jahren, bei denen die Gefahr von Durchblutungsstörungen mit steigendem Alter bekanntlich zunimmt. Ebenso wird die Thrombosegefahr, die in jüngster Zeit eine erhebliche Bedeutung bei der kontrazeptiven Therapie erlangt hat, vermindert.

Überraschenderweise wurde auch festgestellt, daß bei der Verabreichung des erfindungsgemäßen Mittels zuverlässig eine kontinuierliche Unterdrückung des Menstruationszyklus und der Monatsblutung bei ausgesprochen niedriger Dosierung möglich ist. Ohne im folgenden darauf festgelegt werden zu wollen, scheint die Kombination der genannten beiden Hormonkomponenten, und insbesondere die geringe Dosierung der Ethinylestradiols darin, geeignet, die sonst üblichen Nebenwirkungen des Ethinylestradiols zu beseitigen und die ansonsten bei Kontrazeptiva nach dem Stand der Technik typischerweise erforderlichen Gaben von mehr als 15 µg Ethinylestradiol zu unterschreiten.

Die niedrige Dosierung der beiden Hormonkomponenten, und insbesondere der Estrogenkomponente, wird durch die additive Wirkung der beiden Hormonkomponenten ermöglicht, ohne daß es zu einer Beeinträchtigung der Wirkung des erfindungsgemäßen Mittels hinsichtlich seiner kontrazeptiven sowie ovulationshemmenden Eingenschaften kommt.

Die durch das erfindungsgemäße Mittel zuverlässig gewährleistete Ovulationshemmung und Unterdrückung des Menstruationszyklus ist für bestimmte Patientinnen von großer Bedeutung, wie z.B. für Spitzensportlerinnen, Tänzerinnen, und Geschäftsfrauen, die die Beeinträchtigung ihrer körperlichen, geistigen sowie emotionalen Leistungsfähigkeit durch den Menstruationszyklus ausschließen wollen. Infolge der kombinierten und kontinuierlichen Verabreichung der beiden Hormonkomponenten des erfindungsgemäßen Mittels ist es möglich, dieses entweder oral, transdermal, durch Depot-Injektionen oder Hormonimplantate, zu verabreichen. Dabei können auch im vorliegenden Fall die für die jeweiligen Applikationsformen beobachteten Vorteile realisiert werden.

Als orale Darreichungsformen sind all im Stand der Technik bekannten Formen wie beispielsweise Tabletten, Dragees, Pillen oder Kapseln möglich, die unter Verwendung der üblichen Hilfs- und Trägerstoffe hergestellt werden.

Bei der transdermalen Verabreichung des erfindungsgemäßen Mittels können die beiden das Mittel ausbildenden Hormonkomponenten beispielsweise auf ein Pflaster aufgebracht werden oder auch mittels transdermaler therapeutischer Systeme appliziert und somit dem Organismus zugeführt werden, wobei beispielsweise eine bereits vorgefertigte Kombination der beiden Hormonkomponenten oder diese einzeln in ein derartiges System eingebracht werden, welches auf Iontophorese oder Diffusion oder ggf. einer Kombination dieser Effekte beruht.

Für den Fall der oralen Applikation hat es sich als sinnvoll erwiesen, daß Tageseinheiten, die jeweils eine Kombination der beiden Hormonkomponenten umfassen, räumlich getrennt und einzeln entnehmbar in einer Verpackungseinheit angeordnet sind, so daß ein leichte Kontrolle möglich ist, ob tatsächlich die typischerweise tageweise einzunehmende orale Zubereitungsform bereits eingenommen wurde oder nicht. Wichtig ist dabei, daß gewährleistet wird, daß keine einnahmefreien Tage auftreten können. Depot-Injektionen können im Abstand von 1 bis 6 Monaten oder noch länger appliziert werden; Hormonimplantate enthalten die beiden Hormonkomponenten und geben diese über die Dauer von vorzugsweise 3 bis 6 Monaten ab.

Die Menge der verabreichten Gestagene und Estrogene, soweit nicht in Patentanspruch 1 definiert, entspricht im wesentlichen der Menge vergleichbarer Präparate nach dem Stand der Technik. Weitere Angeben betreffend die - täglich - zu verabreichenden Mengen verschiedener, die erste bzw. zweite Hormonkomponente ausbildender Verbindungen finden sich in den Beispielen.

Nachstehend wird die Erfindung anhand von Ausführungsbeispielen weiter erläutert.

### Beispiel 1:

Zur kontrazeptiven Behandlung wurde ein Mittel verwendet, welches pro in Tablettenform vorliegender Tageseinheit 5 µg Ethinylestradiol und 2 mg Norethisteronacetat enthielt. Dabei ist beachtlich, daß Norethisteronacetat in einem Konzentrationsbereich von 0,5 - 5 mg zur Anwendung gelangen kann. Das Mittel wurde über 9 Monate verabreicht und zeigte eine sehr gute kontrazeptive Sicherheit unter vollständiger Unterdrückung des Menstruationszyklus und praktisch keine Nebenwirkungen. Im Rahmen der hier vorgestellten Untersuchung wurde sichergestellt, daß die Probandinnen das Mittel täglich, d.h. ohne Einnahmepause, über den gesamten obengenannten Zeitraum einnahmen.

### Beispiel 2:

Zur kontrazeptiven Behandlung wurde ein Mittel verwendet, welches pro in Tablettenform vorliegender Tageseinheit 0,5 mg Estriol und 2 mg Chlormadinoacetat enthielt. Dabei ist beachtlich, daß Estriol in einem Konzentrationsbereich von 0,5 - 3 mg und Chlormadinonacetat in einem Konzentrationsbereich von 0,75 - 5 mg zur Anwendung gelangen kann. Das Mittel wurde ohne Einnahmepause über 12 Monate verabreicht. Die Wirkungsweise entsprach derjenigen von Beispiel 1.

### Beispiel 3:

Zur kontrazeptiven Behandlung wurde ein Mittel verwendet, welches pro in Tablettenform vorliegender Tageseinheit 0,5 mg Estradiolvalerat und 2 mg Lynestrenol enthielt. Dabei ist beachtlich, daß Estradiolvalerat in einem Konzentrationsbereich von 0,5 - 5 mg und Lynestrenol in einem Konzentrationsbereich von 0,5 - 4,5 mg zur Anwendung gelangen kann. Das Mittel wurde ohne Einnahmepause über 12 Monate verabreicht. Die Wirkungsweise entsprach derjenigen von Beispiel 1.

### Beispiel 4:

Zur kontrazeptiven Behandlung wurde ein Mittel verwendet, welches pro in Tablettenform vorliegender Tageseinheit 7,5 µg Ethinylestradiol und 75 µg Desogestrel enthielt. Dabei ist beachtlich, daß Desogestrel in einem Konzentrationsbereich von 50 - 200 µg zur Anwendung gelangen kann. Das Mittel wurde ohne Einnahmepause über 12 Monate verabreicht. Die Wirkungsweise entsprach derjenigen von Beispiel 1.

### Beispiel 5:

Zur kontrazeptiven Behandlung wurde ein Mittel verwendet, welches pro in Tablettenform vorliegender Tageseinheit 20 mg Tamoxifen und 2 mg Lutenyl enthielt. Dabei ist beachtlich, daß Tamoxifen in einem Konzentrationsbereich von 10 - 50 mg und Lutenyl in einem Konzentratiorisbereich von 1 - 5 mg zur Anwendung gelangen kann. Dieses Mittel ist vorzugsweise geeignet für eine Kontrazeption bei Frauen mit familiärem Brustdrüsenkrebsrisiko. Das Mittel wurde ohne Einnahmepause über 12 Monate verabreicht, die Wirkungsweise entsprach derjenigen von Beispiel 1.

### Beispiel 6:

Zur kontrazeptiven Behandlung wurde ein Mittel verwendet, welches pro in Tablettenform vorliegender Tageseinheit 50 mg Raloxifen und 2,5 mg Medroxyprogesteronacetat (MPA) enthielt. Dabei ist beachtlich, daß Raloxifen in einem Konzentrationsbereich von 30 - 100 mg und Medroxyprogesteronacetat in einem Konzentrationsbereich von 2 - 10 mg zur Anwendung gelangen kann. Diese Kombination eignet sich vorzugsweise bei Frauen mit familiärem Brustdrüsenkrebsrisiko und jungen Frauen nach durchgemachten Brustdrüsenkrebs. Das Mittel wurde ohne Einnahmepause über 12 Monate verabreicht, die Wirkungsweise entsprach derjenigen von Beispiel 1.

### Beispiel 7:

Zur kontrazeptiven Behandlung wurde ein Mittel verwendet, welches pro in Tablettenform vorliegender Tageseinheit 10 µg Ethinylestradiol und Tibolon in einer Konzentration von 2mg täglich enthält. Dabei ist beachtlich, daß Tibolon in einer Konzentration von 1 - 10 mg zur Anwendung gelangen kann. Das Mittel wurde ohne Einnahmepause über 12 Monate verabreicht. Die Wirkungsweise entsprach derjenigen von Beispiel 1.

### Beispiel 8:

Zur kontrazeptiven Behandlung wurde ein Mittel verwendet, welches pro in Tablettenform vorliegender Tageseinheit 10 µg Ethinylestradiol enthält und als Antigestagen die Substanz Ro486 in einer Konzentration von 2,5 mg. Dabei ist beachtlich, daß Ro486 in einem Konzentrationsbereich von 1 - 7,5 mg zur Anwendung gelangen kann. Das Mittel wurde ohne Einnahmepause über 12 Monate verabreicht, die Wirkungsweise entspricht derjenigen von Beispiel 1.

### Beispiel 9 (nachgebracht):

Zur kontrazeptiven Behandlung wurde ein Mittel verwendet, welches pro in Tablettenform vorliegender Tageseinheit 20 µg Ethinylestradiol und 90 µg Levonorgestrel enthielt. Das Mittel wurde über 9 Monate verabreicht und zeigte eine sehr gute kontrazeptive Sicherheit unter vollständiger Unterdrückung des Menstruationszyklus. Nebenwirkungen wurden nicht beobachtet. Es wurde im übrigen sichergestellt, daß die Probandinnen das Mittel ohne Einnahmepause über den gesamten vorgenannten Zeitraum täglich einnahmen.

### Beispiel 10 (nachgebracht):

Zur kontrazeptiven Behandlung wurde ein Mittel verwendet, welches pro in Tablettenform vorliegender Tageseinheit 15 µg Ethinylestradiol und 60 µg Levonorgestrel enthielt. Das Verabreichungsschema entsprach Beispiel 9. Auch hier wurden im wesentlichen dieselben Wirkungen wie bei Beispiel 9 erzielt.

Die in der vorstehenden Beschreibung und in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verwendung einer Kombination aus Levonorgestrel und Ethinylestradiol als alleinigen hormonellen Wirkstoffen zur Herstellung eines Kontrazeptionsmittels zur kontinuierlichen Ovulationshemmung und Unterdrückung des Menstruationszyklus durch ununterbrochene orale, transdermale oder Depot-Verabreichung.

2. Verwendung nach Anspruch 1, **dadurch gekennzeichnet, daß** als Estrogen Ethinylestradiol in einer täglichen Dosis von 5 - 10 µg verwendet wird.

3. Verwendung nach Anspruch 2, **dadurch gekennzeichnet, daß** das Kontrazeptionsmittel zur oralen Verabreichung bestimmt ist.

4. Verwendung nach Anspruch 2 oder 3, **dadurch gekennzeichnet, daß** der bestimmungsgemäße Verabreichungszeitraum neun Monate beträgt.

5. Verwendung nach Anspruch 4, **dadurch gekennzeichnet, daß** der bestimmungsgemäße Verabreichungszeitraum zwölf Monate beträgt.

6. Verwendung der hormonellen Wirkstoffkombination nach einem der vorangehenden Ansprüche zur Herstellung eines Mittels zur Prophylaxe von Osteoporose.

7. Verwendung der hormonellen Wirkstoffkombination nach einem der Ansprüche 1 bis 5 zur Herstellung eines Mittels zur Behandlung des prämenstruellen Syndroms.
